# EUROPÄISCHE PATENTANMELDUNG

(11) **EP 3 018 163 A1**
(43) Veröffentlichungstag der Anmeldung: **11.05.2016**
(21) Anmeldenummer: 14192122.1
(22) Anmeldetag: 06.11.2014
(51) Int. Cl.: C08G 71/04, C07D 317/36

(54) **Neue isocyanatgruppenfreie Polyurethane**

(71) Anmelder: BASF SE, 67056 Ludwigshafen am Rhein (DE)
(72) Erfinder: Mülhaupt, Rolf, 79117 Freiburg (DE); Ritter, Benjamin, 79110 Freiburg (DE); Schmidt, Stanislaus, 79110 Freiburg (DE)
(74) Vertreter: Dr. Langfinger & Partner

(57) **Zusammenfassung**

Isocyanatgruppenfreie Polyurethane erhältlich durch Umsetzung von hydroxylgruppenfreien Monomeren mit einem Strukturelement der allgemeinen Formel II mit mindestens zwei cyclischen Carbonatgruppen 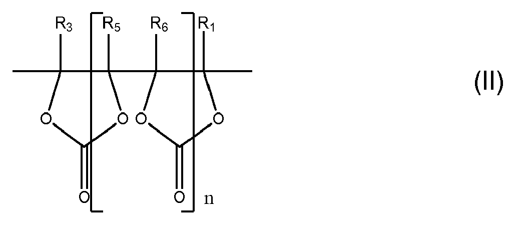 wobei n mindestens 1, R₁, R₃, R₅ und R₆, unabhängig voneinander, ein Wasserstoffatom oder eine Alkylgruppe darstellen, mit einem Polyamin, welches mindestens zwei primäre oder sekundäre Aminogruppen aufweist.

## Beschreibung

Die vorliegende Erfindung betrifft neue isocyanatgruppenfrei hergestellte Polyurethane und Monomere zu deren Herstellung.

Polyurethane sind Polymere, die in großem Maßstab industriell hergestellt und in vielfältigen Anwendungen eingesetzt werden.

Charakteristisches Strukturelement von Polyurethanen ist die Urethanbindung -NH-C(=O)-O- in der Hauptkette des Polymeren. Abhängig von den eingesetzten Reaktanden und den Reaktionsbedingungen können die Eigenschaften über einen weiten Bereich modifiziert werden, woraus sich eine Vielfalt von unterschiedlichen Anwendungsmöglichkeiten ergeben.

Der weitaus größte Teil industriell hergestellter Polyurethane wird durch die Umsetzung von Polyolen mit Polyisocyanaten hergestellt. Die auf diese Weise hergestellten Produkte weisen einen Gehalt an IsocyanatGruppen auf. Isocyanate sind jedoch in der Regel toxikologisch bedenklich und werden häufig unter Verwendung von Phosgen, einer ebenfalls hoch toxischen Substanz hergestellt.

Um das Toxizitätsproblem zu vermeiden, sind isocyanatgruppenfreie und isocyanatfrei hergestellte Polyurethane seit längerem Gegenstand von Forschungsaktivitäten. Wie der Name bereits sagt, werden diese Polyurethane ohne Verwendung von Isocyanaten hergestellt und enthalten daher im Endprodukt keine Isocyanatgruppen.

So wurde die Herstellung von Polyurethanen durch Umsetzung von Polycarbonaten mit Polyaminen in der Literatur mehrfach beschrieben.

In dem Übersichtsartikel von H. Blattmann et al., Isocyanate- and Phosgene-Free Routes to Polyfunctional Cyclic Carbonates and Green Polyurethanes by Fixation of Carbon Dioxide (Macromol. Rapid Commun., 2014, 35 (14), 1238-1254DOI: 10.1002/marc.201400209), wird ein guter Überblick über verschiedene Synthesewege zur Herstellung isocyanatfrei hergestellter Polyurethane gegeben.

In der US 3,084,140 werden Poly-(β-hydroxy-urethane) durch Umsetzung aliphatischer Polyamine mit Carbonaten der allgemeinen Formel I erhalten

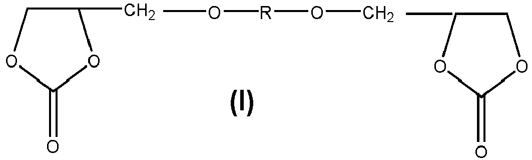

In der jüngeren Vergangenheit wurde zunehmend versucht, die Monomere bei der Herstellung isocyanatgruppenfreier Polyurethane zumindest teilweise aus nachwachsenden, natürlichen Rohstoffen zu gewinnen.

So werden in der US 7,045,577 isocyanatgruppenfreie Polyurethane beschrieben, die durch Umsetzung von cyclische Carbonatgruppen enthaltenden Monomeren, die aus natürlichen Rohstoffen gewonnen werden, mit Diaminen hergestellt werden.

In der WO 2012/171659 werden Terpene und Terpenoide mit mindestens zwei cyclischen Carbonatgruppen beschrieben, die unter anderem zur Herstellung von isocyanatgruppenfreien Polyurethanen eingesetzt werden.

Die aus der Literatur bekannten isocyanatgruppenfreien bzw. isocyanatgruppenfrei hergestellten Polyurethane sind in Ihren Anwendungseigenschaften noch nicht in vollem Umfang zufriedenstellend. So bedarf es meist relativ hoher Temperaturen um eine ausreichende Vernetzung zu erreichen, die für viele Anwendungen gewünscht ist. Auch Quellungseigenschaften und Vernetzungsdichte sind häufig verbesserungsbedürftig.

Der vorliegenden Erfindung lag daher die Aufgabe zugrunde, isocyanatgruppenfreie Polyurethane zur Verfügung zu stellen, die die vorstehend geschilderten Nachteile zumindest teilweise überwinden und damit diesen Produkten breite Anwendungsbereiche erschließen können.

Diese Aufgabe wird erfindungsgemäß mit den isocyanatgruppenfreien Polyurethanen gemäß Anspruch 1 gelöst.

Bevorzugte Ausführungsformen der Erfindung sind den Unteransprüchen und der nachfolgenden detaillierten Beschreibung zu entnehmen. Ebenfalls ist einem Unteranspruch die Synthese solcher cyclischen Carbonate und deren Zwischenprodukte zu entnehmen.

Die isocyanatgruppenfrei hergestellten Polyurethane gemäß der vorliegenden Erfindung sind erhältlich durch Umsetzung von hydroxylgruppenfreien Monomeren mit mindestens zwei cyclischen Carbonatgruppen mit folgendem Strukturelement II im Molekül

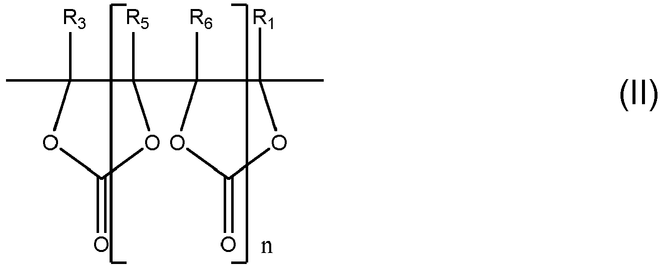

wobei n mindestens 1 und bevorzugt eine ganze Zahl bevorzugt im Bereich von 1 bis 5 ist, jedoch auch größer sein kann, R₁, R₃, R₅ und R₆, unabhängig voneinander, ein Wasserstoffatom oder eine Alkylgruppe, bevorzugt eine C₁-C₆-Alkylgruppe, besonders bevorzugt eine unsubstituierte C₁-C₆-Alkylgruppe darstellen

mit einem Polyamin, welches mindestens zwei reaktive Aminogruppen ausgewählt aus primären und sekundären Aminogruppen, zum Beispiel zwei primäre, eine primäre und eine sekundäre oder zwei sekundäre Aminogruppen, aufweist, wobei die Aminogruppen bevorzugt an primären oder sekundären Kohlenstoffatomen gebunden sind.

Die Reaktivität der cyclischen Carbonate mit dem Strukturelement II geht mit der elektronenziehenden Substitution der zu den cyclischen Carbonatgruppen benachbarten Kohlenstoffatome einher. Deshalb sind sämtliche Substituenten mit elektronenziehender Wirkung geeignet, um die Reaktivität der Carbonatgruppe zu erhöhen und Verbindungen, die cyclische Carbonate enthalten, zu potenziellen Ausgangsverbindungen für raumtemperaturhärtende, isocyanatfrei hergestellte Polyurethane zu machen, solange diese elektronenziehenden Gruppen an den zur cyclischen Carbonatgruppe benachbarten Kohlenstoffatomen bei der Umsetzung mit den Polyaminen nicht mit der Urethanbildungsreaktion konkurrieren oder diese störend beeinflussen. Ester, Amide, Urethane, Carbonate, Harnstoffe als auch Thio- und Phosphorverbindungen des Weiteren auch substituierte als auch unsubstituierte Aromaten und Heteroaromaten können hier als prinzipiell geeignete elektronenziehende Gruppen an den zu den cyclischen Carbonatgruppen benachbarten Kohlenstoffatomen genannt werden.

Bevorzugte Carbonate mit dem Strukturelement II sind Verbindungen der Formel II', in denen R₂ und R₄ unabhängig voneinander H, eine Alkylgruppe, vorzugsweise eine C₁-C₆ Alkylgruppe, die besonders bevorzugt unsubstituiert ist, darstellen und R₁, R₃, R₅ und R₆ die vorstehend definierte Bedeutung haben.

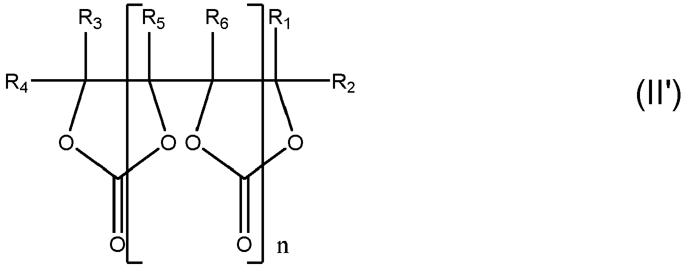

Vorzugsweise sind R₁, R₃, R₅ und R₆ Wasserstoff oder eine C₁-C₆ Alkylgruppe, wie z.B. Methyl. Ethyl, i- oder n-Propyl, i-, n- oder *tert.* Butyl, Pentyl oder Hexyl, besonders sind R₁, R₃, R₅ und R₆ bevorzugt Wasserstoff.

R₂ und R₄ sind vorzugsweise Wasserstoff.

Ganz besonders bevorzugt sind R₁, R₂, R₃, R₄, R₅ und R₆ Wasserstoff.

Hydroxylgruppenfreie Verbindungen der Formel II wobei n eine ganze Zahl im Bereich von 2-5 ist und die Substituenten R₁ bis R₆ wie in Anspruch 1 definiert sind, sind neu und stellen einen weiteren Gegenstand der Erfindung dar.

Beispiele für bevorzugte Verbindungen der allgemeinen Formel II' sind die folgenden Verbindungen

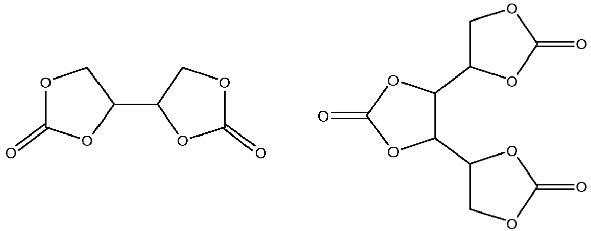

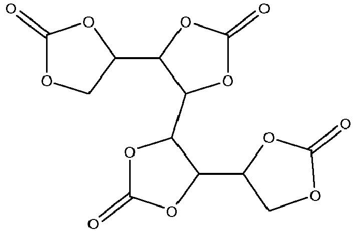

Unter hydroxylgruppenfrei sollen im Rahmen der vorliegenden Erfindung Monomere verstanden werden, die in der Molekülstruktur keine Hydroxylgruppen aufweisen, da diese selbst bei geringen Konzentrationen bei der Umsetzung zu unerwünschten Nebenreaktionen und Nebenprodukten führen können, die sich nachteilig auf die Anwendungseigenschaften der erfindungsgemäßen isocyanatgruppenfreien Polyurethane auswirken und zu erhöhter Vernetzung als auch zu einer erhöhten Wasseraufnahme führen können. Als Polyamine zur Herstellung der erfindungsgemäßen isocyanatfrei hergestelten Polyurethane eignen sich grundsätzlich alle Verbindungen, die mindestens zwei Aminogruppen aufweisen, die mit den cyclischen Carbonatgruppen der hydroxylgruppenfreien Monomere mit dem Strukturelement der Formel II reagieren.

Beispielhaft sind hier zu erwähnen Alkylenamine, Aralkylenamine, Oxyalkylenamine, Haloalkylenamine, Polyoxyalkandiamine oder Polyoxapolyoxyalkandiamine, die vorzugsweise 2-30, insbesondere 2-20 und besonders bevorzugt 3-18 C-Atome und, im Fall der Oxyalkyleneinheiten enthaltenden Polyamine maximal 8, besonders bevorzugt maximal 5 Oxyalkylengruppen aufweisen.

Beispielhaft seien hier erwähnt die isomeren Butandiamine, Pentamethylendiamine, Hexamethylendiamine, Dipropylenglykoldiamin, Triethylenglykoldiamin, Tetrabutylenglykoldiamin, Dipropylentriamin, Tetraethyltriamin, Triethylentetramin, Putrescin und 1,2-Butendiamin, wovon die Alkandiamine wobei insbesondere Ethylendiamin, 1,4-Butandiamin, 1,6-Diaminohexan und das Diamin aus der Dimerfettsäure besonders bevorzugt sind.

Genannt werden können weiterhin 1,2-Propylendiamin, 1,3-Propylendiamin, Neopentandiamin, 1,5-Diamino-2-methylpentan, 2-Butyl-2-ethyl-1,5-pentandiamin, 2,5-Diamino-2,5-dimethylhexan, 2,2,4- und/oder 2,4,4-Trimethyl-1,6-diaminohexan, 1,8-Diaminooctan, 1,11-Diaminoundecan, 1,12-Diaminododecan, 4-Aminomethyl-1,8-octandiamin (Triaminononan), Diethylentriamin, Triethylentetraamin, cycloaliphatische Amine, 2,4- und/oder 2,6-Hexahydrotoluylendiamin, Isopropyl-2,4-diamino-cyclohexan und/oder Isopropyl-2,6-diaminocyclohexan, Tricyclodecan-bis(methylamin), 1,3-Bis-(aminomethyl)-cyclohexan, 2,4'- und/oder 4,4'-Diaminodicyclohexylmethan, 3,3'-Dimethyl-4,4'-diamino-dicyclohexylmethan sowie dessen Isomeren, eine Methylgruppe als Kernsubstituenten aufweisenden Diaminodicyclohexylmethane, 3(4)-Aminomethyl-1-methyl-cyclohexylamin, arylaliphatische Di- oder Triamine, 1,3-Bis-(aminomethyl)-benzol, 3,5-Diethyltoluol-2,4-diamin, *m*-Xylylendiamin. 4,6-Dimethyl-1,3-benzoldimethanamin, 4,4'- und/oder 2,4'- und/oder 2,2'-Methylenbisbenzolamin, Heteroatome enthaltende Amine, die von den Dimerfettsäuren abgeleiteten Diamine und Triamine, Bis-(3-aminopropyl)-methylamin, 4,9-Dioxadodecan-1,12-diamin, 4,7,10-Tri-oxatridecan-1,13-diamin, Zitronensäureamidoamine, Aminoamide, 2,2'-Alkylen-bis-(imidazol-4,5-dihydro)diethanamin, alkoxysilangruppenhaltige Diamine, durch Reaktion bifunktioneller, primärer Amine mit ungesättigte Gruppen erhaltene Michael-Addukte.

Die Reaktionsbedingungen, unter denen die Polyamine mit den Monomeren mit dem Strukturelement der Formel II umgesetzt werden, sind dem Fachmann bekannt und in der Literatur beschrieben, so dass sich hier nähere Angaben erübrigen. Der Fachmann wird, basierend auf der jeweiligen konkreten Situation, die geeigneten Reaktionsbedingungen unter Nutzung seines Fachwissens auswählen.

Über das Molverhältnis von Aminogruppen im Polyamin zu cyclischen Carbonatgruppen im Monomer mit dem Strukturelement der Formel II kann das Molekulargewicht der erhaltenen Produkte gesteuert werden; die höchsten Molekulargewichte werden erhalten, wenn Aminogruppen und cyclische Carbonatgruppen im exakt stöchiometrischen Verhältnis eingesetzt werden.

Bei den isocyanatgruppenfreien Polyurethanen gemäß der vorliegenden Erfindung handelt es sich um Poly-β-hydroxyurethane; bei der Reaktion der Aminogruppen des Polyamins mit den cyclischen Carbonatgruppen entsteht unter Ringöffnung die Urethanbindung sowie eine Hydroxygruppe in β-Stellung gemäß Formel V.

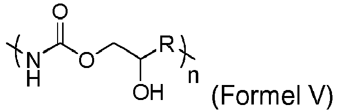

Die Monomere mit dem Strukturelement der allgemeinen Formel II können nach verschiedenen Verfahren hergestellt werden. Wie vorstehend erwähnt, ist die Abwesenheit von Hydroxylgruppen im Molekül von Bedeutung, da sich selbige negativ auf die Eigenschaften der Polyurethane gemäß der Erfindung auswirken. Sollten daher die Monomere mit dem Strukturelement der Formel II aus OH-Gruppen enthaltenden Reaktanden hergestellt werden, ist es wesentlich, dass nicht umgesetzte Mengen an Reaktand quantitativ entfernt werden, da in aller Regel eine quantitative Umsetzung nicht von vornherein sichergestellt ist.

Bevorzugt im Rahmen der vorliegenden Erfindung zur Herstellung der Monomeren mit dem Strukturelement der Formel II werden jedoch Verfahren, die von Reaktanden ausgehen, die frei von OH-Gruppen sind. In diesem Fall stellt sich die Problematik eines Restgehalts an OH-Gruppen von vornherein nicht und es müssen keine aufwendigen Reinigungsschritte unternommen werden. Solange jedoch sichergestellt ist, dass Restgehalte an OH-Gruppen nach der Synthese quantitativ entfernt werden, eignen sich im Prinzip auch Verfahren, die von OH-Gruppen enthaltenden Ausgangsverbindungen ausgehen.

Ein Verfahren, welches von hydroxylgruppenfreien Ausgangsverbindungen ausgeht ist die katalytische Anlagerung von CO₂ an Epoxide, die in der Literatur verschiedentlich beschrieben wurde.

Der Erfolg und die Bedingungen dieser Carbonatisierung hängen von der Art des eingesetzten Katalysators ab. Kohlenstoffdioxid ist ein beinahe inertes Molekül und daher erfordern Reaktionen mit CO₂ eine große Menge Aktivierungsenergie. Aus diesem Grund verläuft diese Carbonatisierung meist bei hohen Drücken und Temperaturen höher 100°C. Der Katalysator stellt meist ein *tetra*-Alkylammoniumhalogenid dar.

Vorzugsweise werden *tetra*-Butyl- oder *tetra*-Ethylammoniumsalze verwendet, da durch die unpolaren Alkylketten in der Regel ein günstigerer Phasentransfer mit organischen Lösungsmitteln erreicht wird. Als Gegenion wird in der Regel Bromid oder Iodid verwendet. Um die Aktivierung des CO₂ zu beeinflussen, können zusätzlich weitere LewisSäuren zum System zugegeben werden, was es ermöglicht, mildere Reaktionsbedingungen anzuwenden. Carbonatisierungen bei Raumtemperatur und 1-2 bar CO₂ Überdruck konnten auf diese Weise durchgeführt werden.

In einigen Fällen wurden gute Ergebnisse mit ungesättigten Epoxiden erzielt; so konnte Butadienmonoxid bei sehr milden Bedingungen unter Verwendung von Palladiumkatalysatoren carbonatisiert werden. In einigen Fällen konnten auch gute Ergebnisse mit Salen-Katalysatoren erzielt werden.

Epoxide der Formel III

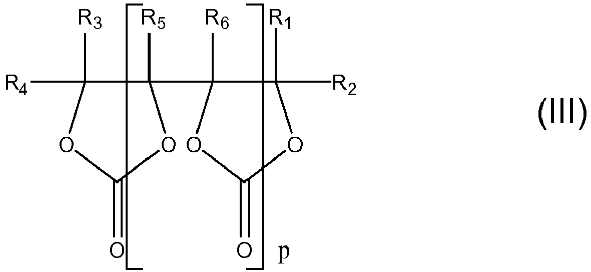

wobei p 0 oder eine ganze Zahl im Bereich von 1 bis 3 ist, R₁, R₃, R₅ und R₆, unabhängig voneinander, ein Wasserstoffatom oder eine Alkylgruppe darstellen und R₂ und R₄ einen Oxiranrest darstellen, sind neu und stellen einen weiteren Gegenstand der vorliegenden Erfindung dar.

Des weiteren betrifft die vorliegende Erfindung ein Verfahren zur Herstellung von Verbindungen der Formel III, in denen p 0, 1 oder 2 ist, durch Epoxidierung von Verbindungen der Formel IV

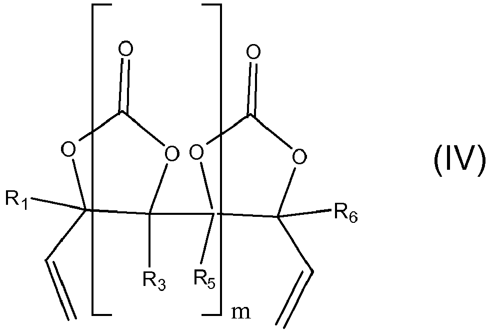

wobei R₁, R₃, R₅ und R₆, unabhängig voneinander, ein Wasserstoffatom oder eine Alkylgruppe darstellen, und m 0 oder 1 ist mit Dimethyldioxiran oder m-Chlorperbenzoesäure.

Die Epoxidierung mit Dimethyldioxiran (DMDO) wird in der Regel in einer Lösung von DMDO (2-10 Gew%) in einem organischen Lösungsmittel, vorzugsweise Aceton, bei einer Temperatur im Bereich von -30 bis 25 °C durchgeführt. Epoxidierungen mit DMDO und die dabei einzuhaltenden Bedingungen sind dem Fachmann an sich bekannt und er wird aufgrund seines Fachwissens die für den jeweiligen Fall passenden Reaktionsbedingungen auswählen.

Besonders bevorzugt als Epoxidierungsmittel im erfindungsgemäßen Verfahren ist m-Chlorperbenzoesäure, die dem Fachmann ebenfalls als Epoxidierungsmittel bekannt ist. Die Umsetzung mit diesem Epoxidierungsmittel wird in der Regel bei Temperaturen zwischen 10 und 80 °C durchgeführt, wobei sich im erfindungsgemäßen Verfahren Temperaturen im Bereich von 30 bis 70 °C besonders bewährt haben.

Das Molverhältnis von Verbindung der Formel IV zu Epoxidierungsmittel liegt bevorzugt im Bereich von 1:1 zu 1:3, ist jedoch im allgemeinen nicht besonders kritisch.

Die Umsetzung wird bevorzugt in Lösung durchgeführt, wobei als Lösungsmittel in bestimmten Fällen chlorierte Lösungsmittel wie Chloroform (Trichlormethan) oder Dichlormethan sich als vorteilhaft erwiesen haben, wovon Dichlormethan besonders bevorzugt wird.

Die Verbindungen der Formel IV können nach dem Fachmann an sich bekannten Verfahren aus leicht zugänglichen Ausgangsverbindungen hergestellt werden und sind teilweise auch kommerziell erhältlich.

Eine beispielhafte Darstellung des Monomeren der Formel II' mit R₁ bis R₆ Wasserstoff und n=2 ausgehend von Glycerin ist nachfolgend dargestellt:

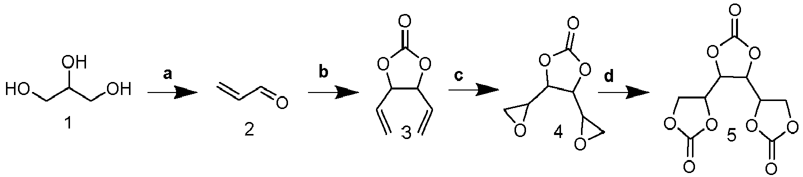

Die Reaktionsschritte a, b und d sind dem Fachmann bekannt und er wird die geeigneten Reaktionsbedingungen aus den in der Literatur beschriebenen Angaben auswählen. Reaktionsschritt c wird nach dem erfindungsgemäßen, vorstehend beschriebenen Verfahren durchgeführt.

Neben der Carbonatisierung von Epoxiden lassen sich Verbindungen mit dem Strukturelement der Formel II auch durch oxidative Carbonatisierung geeigneter Ausgangsverbindungen erhalten. Bei der oxidativen Carbonatisierung wird ein Olefin in einem Schritt zum Oxiran oxidiert, das sofort mit CO₂ zum cyclischen Carbonat umgesetzt wird. Eine Oxidationsmöglichkeit bietet sich durch Luftsauerstoff mit einem Niob-Katalysator, wozu allerdings relativ hohe Drucke und Temperaturen erforderlich sind. Durch Verwendung geeigneter Katalysatoren kann die Umsetzung jedoch auch bei milderen Bedingungen erfolgreich durchgeführt werden.

Die neuen hydroxylgruppenfreien Monomere mit dem Strukturelement der Formel II bzw. die Verbindungen der Formel II' eignen sich hervorragend zur Herstellung isocyanatgruppenfreier Polyurethane. Die auf diese Weise erhältlichen Polyurethane zeichnen sich durch ein interessantes Eigenschaftsspektrum aus und können in vielfältigen Anwendungsbereichen eingesetzt werden.

Insbesondere Monomere mit dem Struktruelement der Formel II, die aus nachwachsenden Rohstoffen (vorzugsweise wegen der besseren Härtbarkeit nicht auf Pflanzenölbasis) erhältlich sind, und die daraus hergestellten isocyanatgruppenfreien Polyurethane stellen zudem eine Alternative zu erdölbasierten Produkten dar. Darüber hinaus kann bei der Herstellung der Monomeren durch Carbonatisierung das Treibhausgas Kohlendioxid reduziert werden, was einen weiteren Vorteil darstellt.

Die erfindungsgemäßen isocyanatfrei hergestellten Polyurethane sind in vielen Fällen bereits bei Raumtemperatur härtend, was einen Vorteil im Vergleich zu isocyanatgruppenfreien Polyurethanen darstellt, die beispielsweise auf Pflanzenöl- oder Glycidyletherbasis hergestellt werden.

Besonders bevorzugte Polyurethane sind die Umsetzungsprodukte von Butadiendicarbonat oder der Verbindung der Formel II' mit n=2 und mit R₁ bis R₆ gleich Wasserstoff mit Alkandiaminen wie z.B. Hexamethylendiamin oder mit von der Dimerfettsäure abgeleiteten Diaminen (die kommerziell in einer großen Zahl im Handel erhältlich sind). Nur beispielhaft sei als Vertreter solcher Dimerfettsäurediamine Priamin® 1074 (erhältlich von Fa. Croda) genannt.

### Beispiele

### Beispiel 1 - Synthese eines zu 100% isocyanatgruppenfreien Polyurethans aus hydroxylgruppenfreiem Sorbitoltricarbonat und Dimerfettsäurediamin (Priamin^{®} 1074, Fa. Croda) bei Raumtemperatur

Sorbitoltricarbonat (1,00g, erhalten nach Beispiel 8)) wurde in Acetonitril (20 ml) gelöst und mit Dimerfettsäurediamin (3,16 g), das in Methanol (10 ml) gelöst wurde, vermischt und für 3 h bei Raumtemperatur gerührt. Das Reaktionsgemisch wurde bei Raumtemperatur einem Rotationsbeschichtungsverfahren ausgesetzt. Als Produkt wurde eine transparente, farblose Schicht erhalten. Mittels Infrarotspektroskopie konnte die vollständige Umsetzung der cyclischen Carbonate bei Raumtemperatur nachgewiesen werden. Die Glasübergangstemperatur (20 K/ min) von Tg = 0°C wurde mittels DSC ermittelt.

### Beispiel 2 - Synthese eines isocyanatgruppenfreien Polyurethans aus hydroxylgruppenfreiem Sorbitoltricarbonat und Butandiamin bei Raumtemperatur

Sorbitoltricarbonat (1,00 g, erhalten nach Beispiel 8) wurde in Acetonitril (20 ml) gelöst und anschließend mit Butandiamin (0,85 g) versetzt. Die Reaktionsmischung wurde bei Raumtemperatur gerührt. Nach 30 min fiel ein unlöslicher farbloser Feststoff aus. Der Feststoff wurde abfiltriert und das Lösungsmittel bei Raumtemperatur entfernt. Mittels Infrarotspektroskopie wurden keine Schwingungen, die dem cyclischen Carbonat zugeordnet werden können, gefunden. Die Glasübergangstemperatur (20 K/ min) betrug 12°C.

### Beispiel 3 - Isocyanatgruppenfreies Polyurethan aus Butadiendicarbonat und Ethylendiamin

Zu Butadiendicarbonat (0,50 g) wurde Ethylendiamin (0.19 g) auf einmal zugegeben. Nach der Zugabe des Diamins wurde die Mischung sofort fest. Das Gemisch wurde mit dem Spatel bei Raumtemperatur gerührt. Das Endprodukt bestand aus kristallinen Körnchen. Mittels Infrarotspektroskopie konnten keine cyclischen Carbonatgruppen identifiziert werden. Die Glasübergangstemperatur (20 K/ min) betrug 40°C.

### Beispiel 4 - Isocyanatgruppenfreies Polyurethan aus hydroxylgruppenfreiem Sorbitoltricarbonat und Dimerfettsäurediamin (Priamin^{®} 1074, Fa. Croda)

Dimerfettsäurediamin (30,0 g) wurde mit Sorbitoltricarbonat (9,79 g, erhalten nach Beispiel 8) bei Raumtemperatur vermischt und die Mischung anschließend mittels Dreiwalzenstuhl homogenisiert. Die Mischung wurde 5 min bei 80°C entgast und in Form gegossen. Die Prüfkörper wurden mit einem Temperaturprogramm von 14 h bei 80°C und 4 h bei 100°C behandelt. Mittels Infrarotspektroskopie konnten keine dem cyclischen Carbonat zuordenbaren Schwingungen im Produkt gefunden werden. Mittels DSC wurde eine Glasübergangstemperatur von 35°C (20 K/ min) ermittelt.

### Beispiel 5 - Isocyanatgruppenfreies Polyurethan aus Butadiendicarbonat und Dimerfettsäurediamin (Priamin^{®} 1074, Fa. Croda)

Butadiendicarbonat ( 10,00 g) wurde unter starkem Rühren zu Dimerfettsäurediamin (31,15 g,) portionsweise zugegeben. Die Suspension wurde anschließend 2 Stunden bei 100 °C gerührt. Das feste Produkt zeigte im FTIR keine Carbonatfunktionen. Der Glasübergang vonT_{g} = -5°C (20 K/ min) konnte mittels DSC ermittelt werden.

### Beispiel 6 - Synthese eines Sorbitoltricarbonat-Dodecandiamin-Prepolymers

Dodecandiamin (23,6 g) wurde bei 100°C geschmolzen. Dazu wurde Sorbitoltricarbonat (2,0 g, erhalten nach Beispiel 8) hinzugegeben und 15 min bei 100°C gerührt. Die Reaktionsmischung wurde abgekühlt. Das Produkt war ein farbloser Feststoff. Die Schmelztemperatur betrug 60°C. Durch Infrarotspektroskopie konnten keine Signale cyclischer Carbonate gefunden werden.

### Beispiel 7 - Synthese eines Butadiendicarbonat-Hexamethylendiamin - Prepolymeren

Hexamethylendiamin (4 g) wurde auf 100°C erwärmt und mit Butadiendicarbonat (1 g) versetzt. Nach 15 min. wurde die Reaktion durch Abkühlen beendet. Das restliche Hexamethylendiamin wurde im Vakuum bei 100°C abgetrennt. Das Produkt wurde als kristalliner Feststoff erhalten. Die Struktur konnte mittels ¹H-NMR Spektroskopie und Infrarotspektroskopie identifiziert werden. 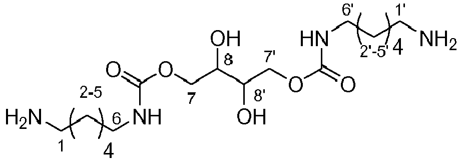 ¹H-NMR(300 MHz, Essigsäure-d₄): v = 1.3 (s, 3,4,3',4'C-*H*₂), 1.6 (s, 2,2',5,5' C-*H*₂), 3.15-3.25 (br, s, 1,1',6,6' C-*H*₂), 3.75-4.0 (8,8'C-*H*), 4.1-4.25 ppm (7,7' C-*H*₂)

### Beispiel 8 -Synthese des Monomeren der Formel II mit R₁ bis R₆ Wasserstoff und n =2.

4,5-Diepoxy-2-yl-1,3-dioxolan-2-on (1,0 g, 28 mmol) wurde in DMSO gelöst und in einem Autoklaven mit 0,02 g (60 mmol) Tetrabutylammoniumbromid (TBAB) versetzt. Der Reaktor wurde dreimal mit CO₂ gespült und ein Druck von 3 MPa CO₂ und eine Temperatur von 80°C eingestellt. Nach 24 h wurde das Reaktionsgemisch auf Raumtemperatur abgekühlt und mit 10 ml destilliertem Wasser versetzt. Der ausgefallene bräunliche Feststoff wurde abfiltriert und mit Wasser (10 ml) und kaltem Aceton (5 ml) gewaschen. Das getrocknete Produkt (200 mg, 29 %) wurde als beigefarbenes Pulver (Schmelzpunkt 230 °C) erhalten. Die Zersetzungstemperatur des Produkts betrug 242 °C. Mittels ¹H-NMR und ¹³C-NMR konnte das Produkt identifiziert werden.

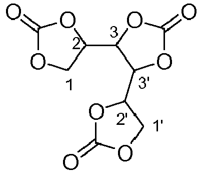

¹H -NMR (300 MHz, Aceton-d₆): v = 4.49 (dd, 2H, 1,1' C-*H*₂), 4.53-4.7 (t, 2H, 1,1' C-*H*₂), 5.1 (s, 2H, 2,2' C-*H*), 5.25 ppm (dd, 2H, 3,3' C-*H*).

### Beispiel 9 -Synthese von 4,5-Diepoxy-2-yl-1,3-dioxolan-2-on

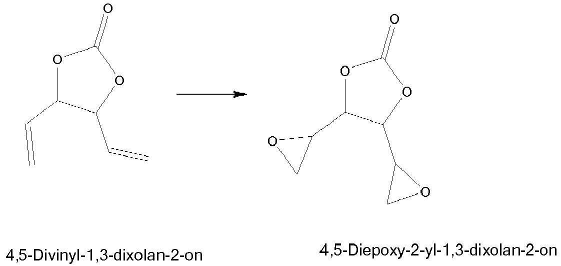

4,5-Divinyl-1,3-dioxolan-2-on (12 g,) wurde in Dichlormethan (120 ml) gelöst und auf O°C abgekühlt. Dazu wurde portionsweise *meta-*Chlorperbenzoesäure (77%, 42,16g) zugegeben. Die Reaktionsmischung wurde drei Tage bei 60°C refluxiert. Die ausgefallene meta-Chlorbenzoesäure wurde abfiltriert. Die Mutterlauge wurde mehrfach über Isohexan extrahiert und das Lösungsmittel am Ende entfernt. Das Produkt (92 %) wurde als eine gelbliche hochviskose Flüssigkeit erhalten. Mittels ¹H-NMR und ¹³C-NMR Spektroskopie konnte das Produkt nachgewiesen werden.

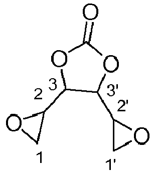

¹H -NMR (300 MHz, CDCl₃): v = 2.9-3.1 (m, 4H, 1,1'C-*H*₂), 3.3-3.6 (m, 2H, 2,2' C-*H*), 4.7-4.8 ppm (m, 1 H, 3,3' C-*H*).

## Patentansprüche

1. Isocyanatgruppenfreie Polyurethane, die aus der Umsetzung von hydroxylgruppenfreien Monomeren mit einem Strukturelement der allgemeinen Formel II mit mindestens zwei cyclischen Carbonatgruppen, 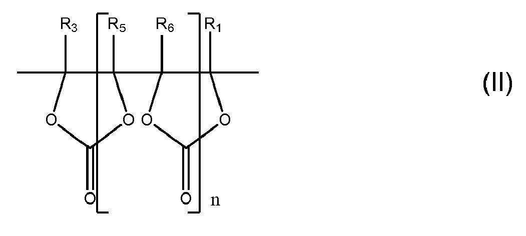 wobei n bevorzugt eine ganze Zahl im Bereich von 1 bis 5 ist, R₁, R₃, R₅ und R₆, unabhängig voneinander, ein Wasserstoffatom oder eine Alkylgruppe darstellen, mit einem Polyamin, welches mindestens zwei primäre oder sekundäre Aminogruppen aufweist, erhältlich sind.

2. Isocyanatgruppenfreie Polyurethane nach Anspruch 1 wobei R₁, R₃, R₅ und R₆ Wasserstoff oder eine C₁-C₆ Alkylgruppe sind.

3. Isocyanatgruppenfreie Polyurethane nach Anspruch 1 oder 2, wobei die hydroxylgruppenfreien Monomere mit dem Strukturelement II der allgemeinen Formel II' entsprechen 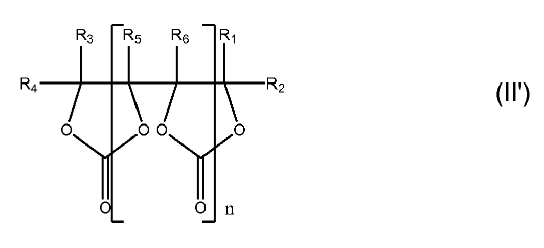 wobei R₂ und R₄ unabhängig voneinander Wasserstoff oder eine Alkylgruppe darstellen und R₁, R₃, R₅ und R₆ die in Anspruch 1 genannte Bedeutung haben.

4. Isocyanatgruppenfreie Polyurethane nach Anspruch 3 wobei R₂ und R₄ Wasserstoff sind.

5. Isocyanatgruppenfreie Polyurethane nach einem der Ansprüche 1 bis 4, wobei das Monomer mit dem Strukturelement der Formel II eine der Verbindungen 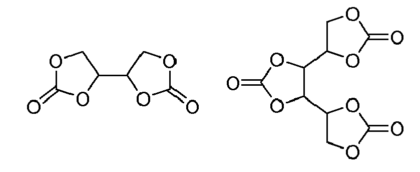 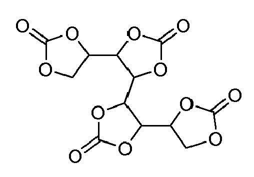 ist.

6. Isocyanatgruppenfreie Polyurethane nach einem der Ansprüche 1 bis 5 wobei das Polyamin ausgewählt ist aus Alkylaminen, Alkenylaminen, Alkylenaminen, Aralkylaminen, Oxyalkylaminen, Haloalkylaminen, Polyoxyalkandiaminen, Aminoalkohol oder Polyoxapolyoxyalkanaminen.

7. Verbindungen der allgemeinen Formel II', wobei n eine ganze Zahl im Bereich von 2-5 ist und die Substituenten R₁ bis R₆ wie in Anspruch 3 definiert sind. Hydroxylgruppenfreie Verbindungen der Formel

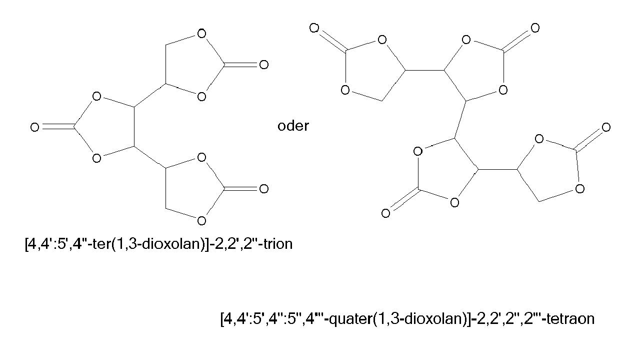

8. Verbindungen der Formel 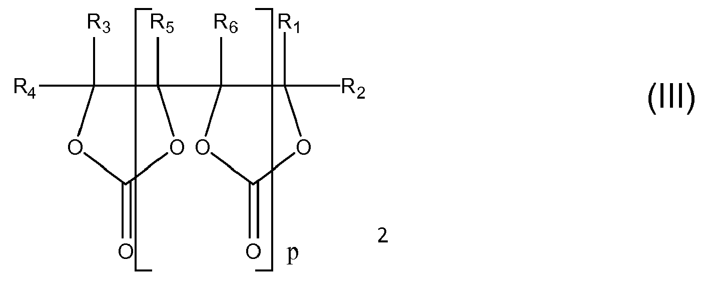 wobei p 0 oder eine ganze Zahl im Bereich von 1 bis 3 ist, R₁, R₃, R₅ und R₆, unabhängig voneinander, ein Wasserstoffatom oder eine Alkylgruppe darstellen und R₂ und R₄ einen Oxiranrest darstellen.

9. Verfahren zur Herstellung von Verbindungen der Formel III mit p= 1 oder 2 durch Epoxidierung von Verbindungen der Formel IV 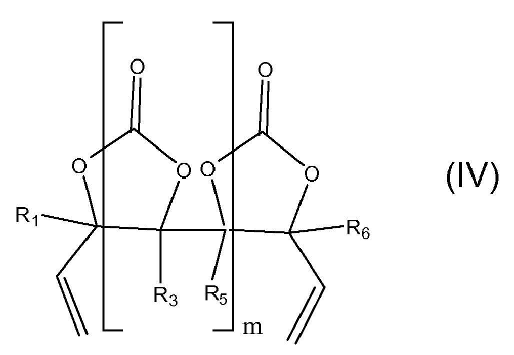 wobei R₁, R₃, R₅ und R₆, unabhängig voneinander, ein Wasserstoffatom oder eine Alkylgruppe darstellen, und m 0 oder 1 ist
mit Dimethyldioxiran oder m-Chlorperbenzoesäure.

10. Verwendung von Monomeren mit einem Strukturelement der Formel II wie in Anspruch 1 definiert zur Herstellung von isocyanatgruppenfreien Polyurethanen.
